# EUROPEAN PATENT SPECIFICATION

(11) **EP 2 980 586 B1**
(45) Date of publication and mention of the grant of the patent: **16.08.2017**
(21) Application number: 14775665.4
(22) Date of filing: 31.03.2014
(51) Int. Cl.: G01N 33/74, G01N 33/543

(54) **INSULIN ASSAY METHOD**
INSULINTESTVERFAHREN
PROCÉDÉ DE TEST DE L'INSULINE

(30) Priority: 29.03.2013 JP 2013074793
(43) Date of publication of application: 03.02.2016
(73) Proprietor: Sekisui Medical Co., Ltd., Tokyo 103-0027 (JP)
(72) Inventor: KONDOU, Junichi, Tokyo 1030027 (JP); NIIYAMA, Kanami, Tokyo 1030027 (JP); YAMAMOTO, Mitsuaki, Tokyo 1030027 (JP)
(74) Representative: Hoffmann Eitle
(86) International application number: PCT/JP2014/059548
(87) International publication number: WO 2014/157723

(56) References cited:
- WO-A1-2011/010673
- JP-A- 2000 292 424
- JP-A- 2001 235 465
- JP-A- 2005 351 643
- JP-A- 2007 121 204

## Description

### Technical Field

The present invention relates to a human insulin latex agglutination immunoassay and assay reagents with which nonspecific reaction in a blood sample assay can be suppressed.

### Background Art

Human insulin, a peptide with a molecular weight of 5807, is one of the important molecular markers for the diagnosis and treatment of diabetes. For reasons related to concentration, competitive RIA and chemiluminescent immunoassay have long been the mainstream immunoassay techniques for an assay of insulin in a human blood sample. With the recent advent of high-sensitivity latex turbidimetric immunoassay (LTIA), LTIA reagents applicable to biochemical auto-analyzer have been reported, and some are commercially available.

The technique described in Non Patent Literature 1 is a sandwich LTIA that uses anti-insulin antibody-immobilized latex particles. However, there are only a few cases of sandwich LTIA used in actual peptide assay applications, and the technique involves problems that need to be solved.

### Citation List

### Patent Literature

PTL 1: WO2011/010673

JP 2001 235465 A discloses a method for determining insulin.

JP 2005 351643 A provides a reagent for a latex turbidimetric immunoassay (LTIA) which decreases the spontaneous aggregation propensity of the particles.

### Non Patent Literature

NPL 1: Kanto Kagaku Cias Insulin II Attachment (created February 2010)

### Summary of Invention

### Technical Problem

The present inventors studied methods of suppressing nonspecific reaction in sandwich LTIA by using specimens that were screened on the basis of the deviation of measurement values obtained with the method of Non Patent Literature 1 from values measured in a heterogeneous chemiluminescent enzyme immunoassay of related art (conventional method), and found that the extent of the deviation from values measured with the conventional method can be reduced when an LTIA immunoreaction is performed with a buffer containing two or more specific pH buffering agents. The present invention was completed on the basis of this finding.

### Solution to Problem

Specifically, the present invention is concerned with the following.
[1] An in vitro insulin assay for performing an immunoreaction with anti-insulin antibody-immobilized particles in a buffer containing two or more amine compounds having a pH buffering effect, wherein the total concentration of said two or more amine compounds having a pH buffering effect is from 100 mM to 1000 mM.
[2] The assay according to [1], wherein the amine compounds having a pH buffering effect are selected from the group consisting of HEPES, MES, and Tris.
[3] The assay according to [1] or [2], wherein the buffer has a pH of 7.3 to 7.8.
[4] A method of suppressing a nonspecific reaction derived from a blood sample in a particle agglutination immunoassay of insulin, wherein the method comprises performing an immunoreaction with anti-insulin antibody-immobilized particles in a buffer containing two or more amine compounds having a pH buffering effect, wherein the total concentration of said two or more amine compounds having a pH buffering effect is from 100 mM to 1000 mM.

### Advantageous Effects of Invention

An assay of the present invention enables a human insulin latex agglutination immunoassay while suppressing nonspecific reaction in a blood sample assay. pH buffering agents used in immunoassays are formulated to achieve an optimum pH in antigen-antibody reaction, and it has been common practice to use two pH buffering agents to widen the pH buffering range, or to adjust pH in combination with the main pH buffering agent. However, it is a completely new finding that containing two or more specific pH buffering agents in a buffer would suppress nonspecific reaction.

### Brief Description of Drawings

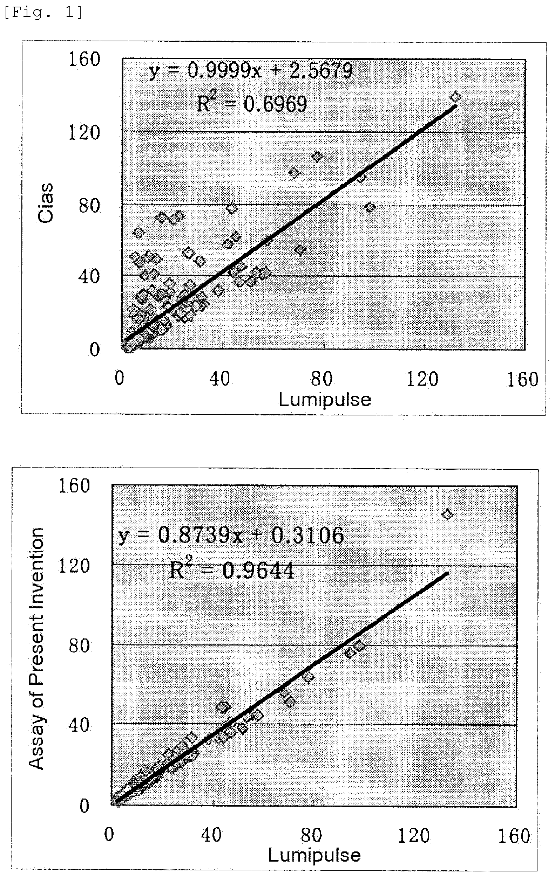

[Fig. 1] Fig. 1 represents correlation diagrams for the measurement results obtained in a conventional LTIA, and the measurement results obtained in the assay of the present invention, in which the upper graph represents the correlation between a chemiluminescent enzyme immunoassay (Lumipulse Presto^{®} Insulin, Fujirebio) and the conventional LTIA (Cias Insulin II, Kanto Kagaku), and the lower graph represents the correlation between the chemiluminescent enzyme immunoassay (Lumipulse Presto^{®} Insulin, Fujirebio) and the assay of the present invention.

### Description of Embodiments

The blood sample used as an analyte in the assay of the present invention is whole blood, serum, or blood plasma.

The buffer containing two or more amine compounds having a pH buffering effect used in the assay of the present invention may be selected from the group consisting of HEPES (2-[4-(2-hydroxyethyl)-1-piperazinyl]ethanesulfonic acid), MES (2-morpholinoethanesulfonic acid), and Tris (tris(hydroxymethyl)aminomethane). The concentration of the buffer (the total concentration of two or more amine compounds having a pH buffering effect when the buffer contains two or more amine compounds having a pH buffering effect) is from 100 mM to 1000 mM, preferably 200 mM to 800 mM, more preferably 400 mM to 800 mM. The preferred pH of the buffer is 7.3 to 7.8.

Anti-insulin antibody-immobilized latex particles usable in the assay of the present invention may be obtained from methods known in the art, including Patent Literature 1.

The reagents used to implement the present invention may have forms of common latex agglutination immunoassay reagents. For example, the reagents may have a form in which the buffer of the present invention is used as a first reagent, and a solution containing anti-insulin antibody-immobilized latex particles is used as a second reagent, or a form of a single reagent in which anti-insulin antibody-immobilized latex particles are contained in the buffer of the present invention.

The reagents used to implement the present invention may contain various known components that can be contained in latex agglutination immunoassay reagents, including, for example, metal ions such as calcium and magnesium ions; ionic, non-ionic, and ampholytic surfactants; chelators such as EDTA; polysaccharides and polymeric compounds such as dextran sulfate; proteins such as albumin; commercially available blocking agents such as Hetero Block (Omega Biologicals, Inc.), Block Ace (DS Pharma), and BPF (Toyobo); and anti-IgM antibodies.

The assay of the present invention may be performed by using the following specific procedure. A blood sample is added to a first reagent primarily composed of pH buffer in an analyzer such as a Hitachi 7170 auto-analyzer, and a second reagent containing anti-insulin antibody-immobilized latex particles is added to the sample mixture after incubating the sample. The sample is then measured for absorbance at appropriate wavelengths, for example, at a main wavelength of 570 nm and a sub wavelength of 800 nm, to determine the insulin concentration.

The concentration of the amine compounds having a pH buffering effect in the assay of the present invention is based on a case where a volume mixture ratio of first reagent to second reagent 3:1.

### Examples

The present invention is described below in more detail using Examples.

### [Example 1]

### 1. Assay Procedures

Sera from 218 patients were measured in a chemiluminescent enzyme immunoassay (Lumipulse Presto^{®} Insulin, Fujirebio), a conventional LTIA (Cias Insulin II, Kanto Kagaku), and the assay of the present invention.

The assay of the present invention was performed with the anti-insulin monoclonal antibody-immobilized latex described in Patent Literature 1, using a first reagent and a second reagent prepared as follows.

### First Reagent

- 600: mM MES
- 200: mM Tris
- 200: mM NaCl
- 50: µg/mL Hetero Block (Omega Biologicals, Inc.)
- 200: µg/mL Anti-IgM antibody
- pH: 7.5

### Second Reagent

- 7.5: mM Tris
- 66221: antibody-immobilized latex
- 66226: antibody-immobilized latex
- pH: 8.0

The serum (10 µL) was added to the first reagent (150 µL) in a Hitachi 7170 auto-analyzer, and a second reagent (50 µL) was added to the mixture after incubating the sample with the first reagent for 5 min at 37°C. The sample was then measured for absorbance at a main wavelength of 570 nm and a sub wavelength of 800 nm at 19 to 34 photometric points over a time course to determine the insulin concentration.

### 2. Assay Results

Among the serum samples measured in the conventional LTIA, 81 samples exhibited the measured values which were in excess of ± 30% of the measured value taken as 100 in the chemiluminescent enzyme immunoassay. On the other hand, none of the sera exceeded ± 30% in the assay of the present invention. The results were checked for correlation by plotting the measured values from the chemiluminescent enzyme immunoassay on X axis, and the measured values from the conventional LTIA or from the assay of the present invention on Y axis. The results are represented in Fig. 1. It can be seen that the deviations from the chemiluminescent enzyme immunoassay results are smaller in the assay of the present invention than in the conventional LTIA.

### Industrial Applicability

The present invention makes it possible to suppress nonspecific reaction in a blood sample assay in a latex agglutination immunoassay of human insulin. This effect of the present invention is based on the selective use of amine compounds commonly used as pH buffering agents, and accordingly, the invention does not impose any unnecessary limitation on the design of reagent formulation. This makes the invention highly useful in industry.

### Reference to Deposited Biological Material

(1) FERM BP-11233
(2) FERM BP-11234

### [Note on Deposited Biological Materials]

### (1) Hybridoma 66221 producing 66221 antibody

- Name and address of depositary where the biological material is deposited
   The National Institute of Advanced Industrial Science and Technology, International Patent Organism Depositary
   Chuo 6, 1-1-1, Higashi, Tsukuba, Ibaraki, Japan (Postal Code 305-8566)
- Date of Deposit of Biological Material in the Depositary
   April 8, 2009 (2009. 4. 8) (Original Deposit Date)
   February 17, 2010 (2010. 2. 17) (Date of Transfer from Original Deposit to Deposit under Budapest Treaty)
- Accession Number Assigned by the Depositary
   FERM BP-11233

### (2) Hybridoma 66226 producing 66226 antibody

- Name and address of depositary where the biological material is deposited
   The National Institute of Advanced Industrial Science and Technology, International Patent Organism Depositary
   Chuo 6, 1-1-1, Higashi, Tsukuba, Ibaraki, Japan (Postal Code 305-8566)
- Date of Deposit of Biological Material in the Depositary
   April 8, 2009 (2009. 4. 8) (Original Deposit Date)
   February 17, 2010 (2010. 2. 17) (Date of Transfer from Original Deposit to Deposit under Budapest Treaty)
- Accession Number Assigned by the Depositary
   FERM BP-11234

## Claims

1. An in vitro insulin assay for performing an immunoreaction with anti-insulin antibody-immobilized particles in a buffer containing two or more amine compounds having a pH buffering effect, wherein the total concentration of said two or more amine compounds having a pH buffering effect is from 100 mM to 1000 mM.

2. The assay according to claim 1, wherein the amine compounds having a pH buffering effect are selected from the group consisting of HEPES, MES, and Tris.

3. The assay according to claim 1 or 2, wherein the buffer has a pH of 7.3 to 7.8.

4. A method of suppressing a nonspecific reaction derived from a blood sample in a particle agglutination immunoassay of insulin, wherein the method comprises performing an immunoreaction with anti-insulin antibody-immobilized particles in a buffer containing two or more amine compounds having a pH buffering effect, wherein the total concentration of said two or more amine compounds having a pH buffering effect is from 100 mM to 1000 mM.

## Patentansprüche

1. In-Vitro-Insulin-Assay zur Durchführung einer Immunreaktion mit Anti-Insulin-Antikörper-immobilisierten Partikeln in einem Puffer, der zwei oder mehr Amin-Verbindungen mit einer pH-Pufferwirkung enthält, wobei die Gesamtkonzentration der zwei oder mehr Amin-Verbindungen mit einer pH-Pufferwirkung 100 mM bis 1000 mM beträgt.

2. Assay gemäß Anspruch 1, wobei die Amin-Verbindungen mit einer pH-Pufferwirkung aus der Gruppe ausgewählt sind, die aus HEPES, MES und Tris besteht.

3. Assay gemäß Anspruch 1 oder 2, wobei der Puffer einen pH-Wert von 7,3 bis 7,8 aufweist.

4. Verfahren zur Unterdrückung einer unspezifischen Reaktion, die von einer Blutprobe stammt, in einem Partikel-Agglutinations-Immunassay von Insulin, wobei das Verfahren das Durchführen einer Immunreaktion mit Anti-Insulin-Antikörper-immobilisierten Partikeln in einem Puffer, der zwei oder mehr Amin-Verbindungen mit einer pH-Pufferwirkung enthält, umfasst, wobei die Gesamtkonzentration der zwei oder mehr Amin-Verbindungen mit einer pH-Pufferwirkung 100 mM bis 1000 mM beträgt.

## Revendications

1. Dosage d'insuline in vitro pour effectuer une immunoréaction avec des particules immobilisées sur un anticorps anti-insuline dans un tampon contenant deux composés d'amine ou plus ayant un effet tampon sur le pH, dans lequel la concentration totale desdits deux composés d'amine ou plus ayant un effet tampon sur le pH est de 100 mM à 1000 mM.

2. Dosage selon la revendication 1, dans lequel les composés d'amine ayant un effet tampon sur le pH sont sélectionnés à partir du groupe constitué par HEPES, MES et Tris.

3. Dosage selon la revendication 1 ou 2, dans lequel le tampon a un pH de 7,3 à 7,8.

4. Procédé de suppression d'une réaction non spécifique obtenue à partir d'un échantillon de sang dans un immunodosage d'insuline par agglutination de particules, dans lequel le procédé comprend l'exécution d'une immunoréaction avec des particules immobilisées sur un anticorps anti-insuline dans un tampon contenant deux ou plusieurs composés d'amine ayant un effet tampon sur le pH, dans lequel la concentration totale desdits deux composés d'amine ou plus ayant un effet tampon sur le pH est de 100 mM à 1000 mM.
